# EUROPÄISCHE PATENTANMELDUNG

(11) **EP 1 344 551 A2**
(43) Veröffentlichungstag der Anmeldung: **17.09.2003**
(21) Anmeldenummer: 03012355.8
(22) Anmeldetag: 01.08.1996
(51) Int. Cl.: A61N 1/04, A61N 1/05

(54) **Mikroelektroden-Anordnung**

(30) Priorität: 10.08.1995 DE 19529371
(62) Teilanmeldung aus: 96931732.0
(71) Anmelder: NMI NATURWISSENSCHAFTLICHES UND MEDIZINISCHES INTITUT AN DER UNIVERSITÄT TÜBINGEN, 72770 Reutlingen (DE)
(72) Erfinder: Nisch, Wilfried, 72070 Tübingen (DE)
(74) Vertreter: Otten, Hajo, Dr.-Ing.

(57) **Zusammenfassung**

Die vorliegende Erfindung betrifft die Verwendung einer Mikroelektroden-Anordnung zur elektrischen Stimulation von Netzwerken biologischer Zellen. Die Mikroelektroden-Anordnung weist eine Vielzahl von Mikroelektroden auf (M₁ bis Mₙ), wobei jede Mikroelektrode (M₁ bis Mₙ) eine Kontaktierelektrode (K₁ bis Kₙ), eine Anschlusselektrode (A₁ bis Aₙ) und ein lichtempfindliches Element (P) aufweist. Das lichtempfindliche Element (P) dient als Schalter, der die Kontaktierelektrode (K₁ bis Kₙ) von der Anschlusselektrode (A₁ bis Aₙ) isoliert oder mit der Anschlusselektrode (A₁ bis Aₙ) verbindet, so dass jede Mikroelektrode (M₁ bis Mₙ) für sich durch Licht ansteuerbar ist.

## Beschreibung

Die Erfindung betrifft eine Mikroelektroden-Anordnung und deren Verwendung zur elektrischen Stimulation von Netzwerken biologischer Zellen.

Biologische Zellen oder Netzwerke aus biologischen Zellen wie z.B. Zellkulturen, Gewebeschnitte "in vitro" oder biologisches Gewebe "in vivo" werden in der Elektrophysiologie üblicherweise durch Glasmikroelektroden mit Elektrolytfüllung oder durch Metallmikroelektroden kontaktiert. Die Elektroden werden mittels eines sog. Mikromanipulators in eine Zelle eingestochen (intrazelluläres Verfahren), mit einer Zellmembran in dichten Kontakt gebracht (patch clamp-Verfahren) oder in die Nähe der Zellmembran gebracht (extrazelluläres Verfahren), so dass die Mikroelektroden elektrisch leitend über eine Elektrolytlösung mit den biologischen Zellen des Netzwerks verbunden sind. Der Nachteil dieser Kontaktier-Verfahren ist, dass nur eine oder mit großem Aufwand nur wenige Zellen gleichzeitig mit Mikroelektroden kontaktiert und infolgedessen keine Netzwerkeigenschaften untersucht werden können.

Aus diesem Grunde wurde in neuerer Zeit versucht, ein Netzwerk aus biologischen Zellen mittels Mikroelektroden, die auf ein Substrat (Träger) mit aus der Mikroelektronik bekannten Methoden aufgebracht und mikrostrukturiert sind, an vielen Stellen gleichzeitig zu kontaktieren, um elektrische Zellpotentiale extrazellulär ableiten oder die Zellen elektrisch stimulieren zu können. Dabei sollen die Mikroelektroden in möglichst hoher Dichte angeordnet sein, um eine hohe örtliche Auflösung zu erzielen. Des weiteren sollen die elektrischen Potentiale der Zellen möglichst gleichzeitig, also parallel, abgeleitet bzw. elektrische Potentiale zur Stimulation des Netzwerks gleichzeitig an dessen Zellen angelegt werden können, um eine hohe zeitliche Auflösung zu erreichen.

Dabei besteht allerdings das Problem, dass elektrische Leitungen von den einzelnen Mikroelektroden isoliert bis zu einer Mess- oder Stimulationselektronik oder dgl. geführt werden müssen. Die Vielzahl voneinander isolierter, paralleler Leitungen begrenzt die örtliche Auflösung der Mikroelektroden-Anordnung.

Eine andere Möglichkeit ist, einen integrierten elektronischen Schalter für jede Mikroelektrode auf dem Substrat unterzubringen und die Mikroelektroden im Multiplexbetrieb einzeln oder in Gruppen zeitlich nacheinander mit der Mess- oder Stimulationselektronik zu verbinden (anzusteuern). Dies erfordert einen sehr hohen Aufwand an integrierter Schaltungstechnik (VLSI Technik) und verteuert dadurch die Mikroelektroden-Anordnung ganz erheblich. Des Weiteren bleibt die örtliche Auflösung wegen der auf dem Substrat unterzubringenden elektronischen Schalter begrenzt. Darüber hinaus können die Mikroelektroden nicht mehr gleichzeitig, sondern nur einzeln oder in Gruppen nacheinander angesteuert werden, die Zeitauflösung der Ableitung oder Stimulation wird herabgesetzt. Weiterer Nachteil sind Störspannungen, die von den elektronischen Schaltern beim Schalten auf die Mikroelektroden und auf deren Anschlussleitungen übertragen werden können und das Messsignal überlagern. Diese Störspannungen verschlechtern das Messergebnis und das Signal/Rauschverhältnis. Die Störspannungen können das Messsignal um ein Vielfaches übersteigen, weswegen ihr Abklingen nach dem Schalten abgewartet werden muss, bevor überhaupt gemessen oder stimuliert werden kann. Dadurch wird die Zeitauflösung der Mikroelektroden-Anordnung weiter herabgesetzt.

Die Anzahl der Mikroelektroden bekannter Mikroelektroden-Anordnungen ist infolgedessen begrenzt (weniger als 100 Mikroelektroden).

Der Erfindung liegt daher die Aufgabe zugrunde, eine Mikroelektroden-Anordnung und eine Verwendung der eingangs genannten Art anzugeben, mit einer sehr großen Anzahl an Mikroelektroden, die durch kleine Abmessungen der Mikroelektroden und Abstände voneinander eine hohe Ortsauflösung und außerdem eine hohe zeitliche Auflösung ermöglicht.

Diese Aufgabe wird durch eine Verwendung mit den Merkmalen des Anspruchs 1 und eine Mikroelektroden-Anordnung mit den Merkmalen des Anspruchs 17 gelöst. Jede Mikroelektrode weist dabei eine Kontaktierelektrode, einen Anschluss für eine Stimulationselektronik oder dgl., im folgenden als Anschlusselektrode bezeichnet, sowie ein lichtempfindliches Element auf.

Die Kontaktierelektrode ist über eine Elektrolytlösung in elektrisch leitenden Kontakt mit einer biologischen Zelle eines Netzwerks bringbar. Dies erfolgt vorzugsweise, indem die Mikroelektroden-Anordnung an ein Netzwerk biologischer Zellen heran und dadurch die Mikroelektroden in unmittelbare Nähe von Zellmembranen gebracht werden, also extrazellulär. Dabei besteht ein elektrischer Übergangswiderstand (Impedanz) zwischen den Zellen und den Mikroelektroden.

Das lichtempfindliche Element, das bei Dunkelheit einen sehr hohen elektrischen Widerstand hat, der sich bei Auftreffen von Licht verringert (oder umgekehrt), ist zwischen der Kontaktierelektrode und der Anschlusselektrode angeordnet und dient als Schalter, der die Kontaktierelektrode von der Anschlusselektrode isoliert oder als ohmscher Widerstand mit der Anschlusselektrode verbindet. Betätigt wird dieser Schalter, indem Licht auf ihn, d.h. auf das lichtempfindliche Element, gerichtet wird. Somit ist jede Mikroelektrode für sich durch Licht ansteuerbar, die Mikroelektroden sind lichtadressierbar.

Die Erfindung hat den Vorteil, dass ihre Mikroelektroden sehr kleine Abmessungen aufweisen und sehr dicht beieinander anordenbar sind, so dass sich eine hohe örtliche Auflösung erzielen läßt. Weiterer Vorteil der Erfindung ist, dass die Mikroelektroden einzeln oder in Gruppen gleichzeitig, d.h. parallel, ansteuerbar sind, was eine hohe zeitliche Auflösung ermöglicht. Weiterer Vorteil ist, dass durch die Ansteuerung mit Licht keine Störspannungen auftreten, die das Messsignal überlagern und deren Abklingen vor einer Messung oder bis zu einer Stimulation abgewartet werden müsste.

Die Kontaktierelektroden, das lichtempfindliche Element und die Anschlusselektroden können in zwei oder drei Ebenen übereinander oder auch in einer Ebene nebeneinander auf einem Substrat angeordnet werden. Dabei ergibt die Anordnung in drei Ebenen übereinander die dichteste Anordnung der Mikroelektroden beieinander und damit die höchste örtliche Auflösung.

Zur Isolation der Kontaktierelektroden und der Anschlusselektroden der verschiedenen Mikroelektroden voneinander kann das lichtempfindliche Element dienen, das vorzugsweise, wenn es nicht mit Licht beaufschlagt wird, also dunkel ist, elektrisch isoliert. Das lichtempfindliche Element ist in diesem Fall als für alle oder für Gruppen von Mikroelektroden gemeinsame, durchgehende Schicht ausgebildet, auf die örtlich auf die anzusteuernden Mikroelektroden begrenzt Licht gerichtet wird. In diesem Fall muss zur Ansteuerung mit Licht entweder die Kontaktierelektrode oder die Anschlusselektrode und das Substrat, auf das die Mikroelektroden aufgebracht sind, lichtdurchlässig sein.

Werden die lichtempfindlichen Elemente neben den Kontaktierelektroden oder neben den Anschlusselektroden angeordnet, so können die Kontaktierelektroden und die Anschlusselektroden lichtundurchlässig, aus demselben Material hergestellt und in einem Arbeitsgang auf das Substrat aufgebracht werden.

Die Anschlusselektroden aller oder von Gruppen der Mikroelektroden können zu einer gemeinsamen Anschlusselektrode vereinigt sein. Dadurch verringert sich die erforderliche Anzahl an Anschlussleitungen, jedoch können die Mikroelektroden nicht mehr parallel, sondern nur seriell bzw. in Gruppen parallel angesteuert werden.

Zur Ansteuerung der Mikroelektroden ist bei einer Ausgestaltung der Erfindung eine Lichtfaseroptik vorgesehen, die vorzugsweise so viele Lichtfasern aufweist, wie die Anordnung Mikroelektroden umfasst, so dass zu jeder Mikroelektrode eine Lichtfaser führt. Dabei können die Stirnenden der Lichtfasern, aus denen das Licht austritt, als Substrat für die Mikroelektroden dienen.

Bei einer Weiterbildung der Erfindung weist die Lichtfaseroptik eine Lichtquelle für jede Lichtfaser auf. Vorzugsweise sind die Lichtquellen zu einer Matrix zusammengefasste Leuchtdioden.

Die erfindungsgemäße Mikroelektroden-Anordnung lässt sich zur Ableitung von Impulsen oder zur elektrischen Stimulation von Nervenzellen in Pflanzen oder Lebewesen implantieren. Beispielsweise ist die erfindungsgemäße Mikroelektroden-Anordnung als Retina-Implantat verwendbar.

Zur Ansteuerung bestimmter Mikroelektroden der erfindungsgemäßen Anordnung findet fokussiertes Licht, beispielsweise ein Laserstrahl Verwendung. Es können Muster aus Lichtpunkten, Lichtbalken oder dergleichen auf die Anordnung projiziert werden, um bestimmte Mikroelektroden gleichzeitig anzusteuern.

Die Erfindung wird nachfolgend anhand eines in der Zeichnung dargestellten Ausführungsbeispiels näher erläutert. Es zeigen:
- Figur 1: einen Schnitt durch eine erfindungsgemäße Mikroelektroden-Anordnung mit seriell (Figur la) bzw. parallel (Figur 1b) anzusteuernden Mikroelektroden;
- Figur 2: eine schematische Darstellung einer erfindungsgemäßen Mikroelektroden-Anordnung (Figur 2a seriell, Figur 2b parallel);
- Figur 3: eine Draufsicht auf eine erfindungsgemäße Mikroelektroden-Anordnung mit spaltenparallel geschalteten und zeilenparallel anzusteuernden Mikroelektroden; und
- Figur 4: einen Schnitt entlang Linie IV-IV in Figur 3.

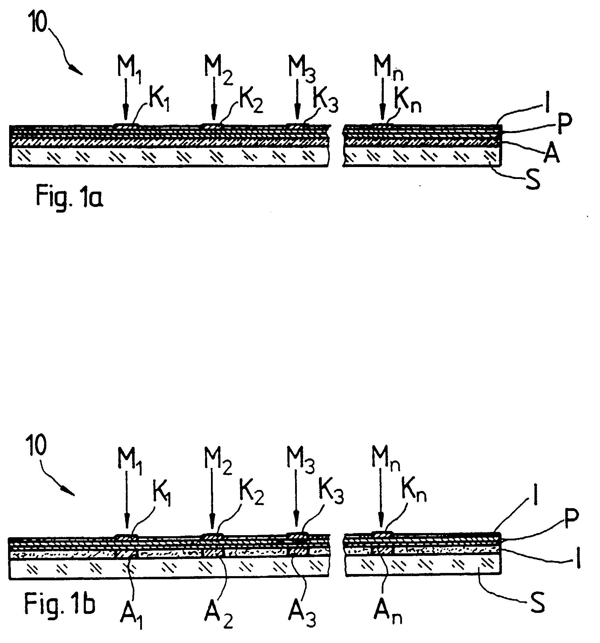

Die in Figuren 1a und b dargestellte, Mikroelektroden-Anordnung 10 ist auf ein Substrat S aufgebracht. Das Substrat S besteht vorzugsweise aus einem lichtdurchlässigen Material, wie z.B. Glas oder Kunststoff. Es kann jedoch auch aus einem lichtundurchlässigen Material wie z.B. Keramik oder Silizium mit Oxidschichtisolator bestehen, die an sich aus der Mikroelektronik bekannt sind.

Die Mikroelektroden M₁ bis Mₙ umfassen Anschlusselektroden A bzw. A₁ bis Aₙ, lichtempfindliche Elemente P und Kontaktierelektroden K₁-Kₙ, die in genannter Reihenfolge in drei Ebenen übereinander als Dünnschichtelemente auf das Substrat S aufgebracht sind. Bei serieller Ansteuerung der Mikroelektroden M₁ bis Mₙ kann eine einzige, durchgehende Anschlusselektrode A für alle Mikroelektroden M₁ bis Mₙ gemeinsam auf das Substrat S aufgebracht sein (Figur 1a). Bei paralleler Ansteuerung weist jede Mikroelektrode M₁ bis Mₙ eine Anschlusselektrode A₁ bis Aₙ auf, die durch eine Isolatorschicht I voneinander getrennt sind. Die Isolatorschicht I ist in einer Ebene mit den Anschlusselektroden A₁ bis An auf das Substrat S aufgebracht.

Die lichtempfindlichen Elemente sind als durchgehende Schicht P für alle Mikroelektroden M₁ bis Mₙ gemeinsam auf die Anschlusselektroden A bzw. A₁ bis An und ggf. die Isolierschicht I aufgebracht. Auf die die lichtempfindlichen Elemente bildende lichtempfindliche Schicht P sind die Kontaktierelektroden K₁ bis Kₙ aufgebracht, die sich bei paralleler Ansteuerung über den Anschlusselektroden A₁ bis Aₙ befinden. Die Kontaktierelektroden K₁ bis Kₙ sind ebenfalls mit einer Isolatorschicht I voneinander getrennt, die in einer Ebene mit den Kontaktierelektroden K₁ bis Kₙ auf die lichtempfindliche Schicht P aufgebracht sind. Die Kontaktierelektroden K₁ bis Kₙ stehen geringfügig über ihre Isolatorschicht I vor.

Die als Dünnschichtelemente ausgebildeten Kontaktierelektroden K₁ bis Kₙ, lichtempfindlichen Elemente P und Anschlusselektroden A bzw. A₁ bis Aₙ werden durch Aufdampfen, Sputtern oder PECVD (Plasma-Enhanced-Chemical-Vapor-Deposition) auf das Substrat S aufgebracht und mit photolithografischen Methoden mikrostrukturiert.

Die Anschlusselektroden A bzw. A₁ bis An bestehen aus einem elektrisch gut leitfähigen, vorzugsweise lichtdurchlässigen Material, wie z. B. Indiumzinnoxid (ITO) oder Zinkoxid (ZnO).

Die als durchgehende Schicht P ausgebildeten, lichtempfindlichen Elemente können als Dünnschicht-Fotowiderstände, Fotodioden mit PN- oder PIN-Übergang oder als Fototransistoren ausgeführt sein, die in Dünnschichttechnologie aus Materialien wie z.B. amorphem Silizium (Si), Cadmiumsulfid (CdS) oder Cadmiumselenid (CdSe) hergestellt sein können.

Die Kontaktierelektroden K₁ bis Kₙ bestehen vorzugsweise aus einem biokompatiblen, leitfähigen Material wie z.B. Gold (Au), Platin (Pt), Titan (Ti), Iridium (Ir) und sind durch die biokompatible Isolatorschicht I aus z.B. Siliziumoxid, Siliziumnitrid oder Polyimid voneinander isoliert. Die Kontaktierelektroden können auch aus lichtdurchlässigem Material, wie es für die Anschlusselektroden A bzw. A₁ bis An Verwendung findet, hergestellt sein. Ebenso können die Anschlusselektroden A bzw. A₁ bis Aₙ lichtundurchlässig aus demselben Material wie die Kontaktierelektroden K₁ bis Kₙ hergestellt sein.

Bei der in Figur 1a dargestellten Ausführungsform der Erfindung ist eine gemeinsame Leitung für alle Mikroelektroden M₁ bis Mₙ zum Anschluss an eine Stimulationselektronik oder dgl. an der gemeinsamen, durchgehend ausgebildeten Anschlusselektrode A, vorzugsweise in deren Randbereich, angebracht (nicht dargestellt). Bei der in Figur 1b dargestellten Ausführungsform der Erfindung mit voneinander isolierten Anschlusselektroden A₁ bis Aₙ weisen diese jeweils eigene Anschlussleitungen auf (nicht dargestellt).

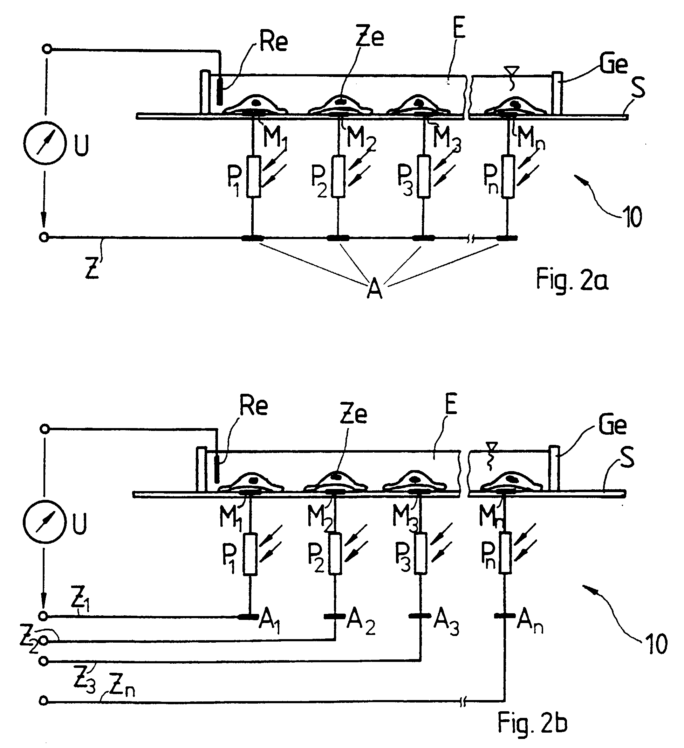

Die schematische Darstellung der Figuren 2a und b zeigt die Anwendung der erfindungsgemäßen Mikroelektroden-Anordnungen 10 aus Figuren 1a und b zur Ableitung elektrischer Zellpotentiale oder zum elektrischen Stimulieren von Netzwerken biologischer Zellen Ze. Die biologischen Zellen Ze befinden sich in einem zylindrischen Kulturgefäß Ge in einem physiologischen Elektrolyten E. Den Boden des Kulturgefäßes Ge bildet das Substrat S mit der Mikroelektroden-Anordnung M₁ bis Mₙ aus Figuren 1a und b. Dabei befinden sich die in Figuren 2a und b nicht im einzelnen dargestellten Kontaktierelektroden K₁ bis Kₙ dicht an Zellmembranen der Zellen Ze und sind dadurch über den Elektrolyten elektrisch leitend mit jeweils einer Zelle Ze verbunden (extrazellulär), wobei ein elektrischer Widerstand (Impedanz) zwischen Zelle Ze und der Kontaktierelektrode K₁ bis Kₙ der jeweiligen Mikroelektrode M₁ bis Mₙ besteht.

In den physiologischen Elektrolyten E ist eine Referenzelektrode Re aus Metall getaucht, so dass ein elektrisches Potential an jeder gewünschten Stelle des Netzwerkes biologischer Zellen Ze mit den Mikroelektroden M₁ bis Mₙ gemessen oder das Netzwerk biologischer Zellen Ze an allen gewünschten Stellen mit den Mikroelektroden M₁ bis Mₙ elektrisch stimuliert werden kann.

Die auf dem Substrat S aufgebrachten lichtempfindlichen Elemente P₁ bis Pₙ und Anschlusselektroden A bzw. A₁ und Aₙ sind in Figuren 2a und b mit ihren Anschlussleitungen Z, Z₁ bis Zₙ in Form eines elektrischen Schaltbildes dargestellt.

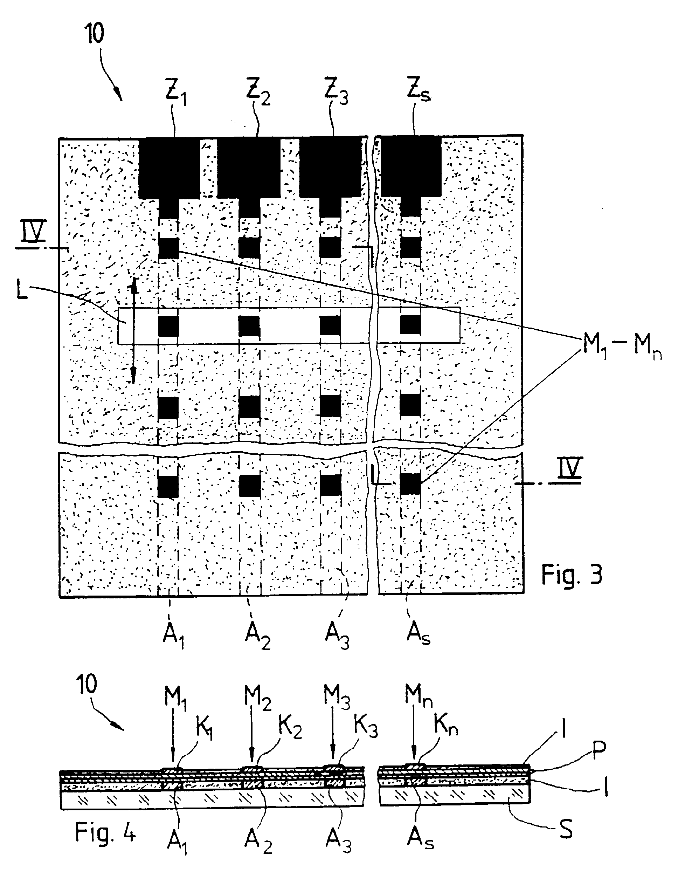

Die Figuren 3 und 4 zeigen eine erfindungsgemäße Mikroelektroden-Anordnung 10 mit spaltenparallel geschalteten Mikroelektroden M₁ bis Mₙ, wobei der Schnitt gemäß Figur 4 den Figuren 1a und b entspricht. Aufbau und Anordnung der Kontaktierelektroden K₁ bis Kₙ, die durch eine Isolatorschicht I voneinander isoliert sind, und die darunter liegende lichtempfindliche Dünnschicht P stimmen mit der oben beschriebenen, in Figuren 1a und b dargestellten Anordnung überein. In Figur 3 ist die matrixförmige Anordnung der Mikroelektroden M₁ bis Mₙ zu sehen. Anschlusselektroden A₁ bis Aₙ sind als parallele, in einer Spaltenrichtung durchgehende Leiterbahnen ausgebildet, die sich an einem Rand des Substrats S zu Kontaktierflächen Z₁ bis Zₛ vergrößern. An den Kontaktierflächen Z₁ bis Zₛ werden nicht dargestellte Anschlusskabel zum Anschluss der Mikroelektroden-Anordnung 10 an eine Mess- oder Stimulationselektronik angelötet, angeschweißt oder auf sonstige, an sich bekannte Weise elektrisch leitend angebracht. Die Mikroelektroden M₁ bis Mₙ sind bei der Ausführungsform gemäß Figuren 3 und 4 zu je eine Spalte umfassenden Gruppen zusammengefasst. Anstelle von Spalten können beispielsweise auch Kreise oder sonstige Gruppen von Mikroelektroden M₁ bis Mₙ zusammengefasst werden.

Die Anschlusselektroden A₁ bis Aₛ sind durch eine Isolatorschicht I voneinander getrennt. Als Materialien für die Kontaktierelektroden K₁ bis Kₙ, die lichtempfindliche Schicht P, die Anschlusselektroden A₁ bis Aₙ, die Isolatorschichten I und das Substrat können dieselben Materialien wie zu Figuren 1a und b aufgeführt Verwendung finden.

Bei der spaltenparallelen Schaltung der Mikroelektroden M₁ bis Mₙ kann jeweils nur eine Mikroelektrode M₁ bis Mₙ jeder Spalte angesteuert, d.h. mit ihr abgeleitet oder stimuliert werden. Die Ansteuerung kann zeilenweise oder auch nach einem anderen Muster erfolgen.

Die Ansteuerung der erfindungsgemäßen Mikroelektroden-Anordnung 10, die nachfolgend anhand Figur 3 erläutert wird, erfolgt mittels eines fokussierten oder geformten Lichtstrahls oder eines projizierten Lichtbildes, das beispielsweise unter Verwendung eines Lasers erzeugt oder mittels Glasfasern den Mikroelektroden M₁ bis Mₙ zugeführt wird. Zur Ansteuerung wird die lichtempfindliche Schicht P im Bereich einer oder mehrerer anzusteuernder Mikroelektroden M₁ bis Mₙ beleuchtet. Der beleuchtete Bereich bildet das lichtempfindliche Element der jeweiligen Mikroelektrode M₁ bis Mₙ. Der beleuchtete Bereich der lichtempfindlichen Schicht P wird elektrisch leitend, so dass die Kontaktierelektroden K₁ bis Kₙ der angesteuerten Mikroelektroden M₁ bis Mₙ elektrisch leitend mit der zugehörigen Anschlusselektrode A₁ bis Aₛ verbunden ist und das elektrische Potential einer in der Nähe der jeweiligen Mikroelektrode M₁ bis Mₙ befindlichen biologischen Zelle (Figuren 2a und b) abgeleitet, d.h. gemessen oder die biologische Zelle elektrisch stimuliert werden kann.

Die Ansteuerung erfolgt entweder mittels Auflicht, d.h. durch das Netzwerk biologischer Zellen hindurch von der Seite der Kontaktierelektroden K₁ bis Kₙ her. In diesem Fall müssen die Kontaktierelektroden K₁ bis Kₙ lichtdurchlässig oder seitlich neben der sie von ihrer Anschlusselektrode A₁ bis Aₛ trennenden, das lichtempfindliche Element bildenden lichtempfindlichen Schicht P angeordnet sein. Ebenso kann die Ansteuerung mit Durchlicht von der Seite des Substrats S her erfolgen. In diesem Fall muss das Substrat S und müssen die Anschlusselektroden A₁ bis Aₛ lichtdurchlässig oder neben der sie von den Kontaktierelektroden K₁ bis Kₙ trennenden, das lichtempfindliche Element bildenden lichtempfindlichen Schicht P angeordnet sein. Im unbeleuchteten Bereich isoliert die Dünnschicht P. Sie bildet also durch örtlich begrenzte Beleuchtung im Bereich einer Mikroelektrode M₁ bis Mₙ im beleuchteten Bereich das lichtempfindliche Element dieser Mikroelektrode M₁ bis Mₙ.

Bei Verwendung von amorphem Silizium werden bis zu fünf Zehnerpotenzen umfassende Widerstandsverhältnisse zwischen beleuchtet (hell) und unbeleuchtet (dunkel) erreicht. Bei einer Mikroelektrode M₁ bis Mₙ mit einer Fläche von 10 µm mal 10 µm und einer Dicke von 0,1 µm ergibt sich bei einer 25 Dunkelleitfähigkeit von Sigma = 10⁻⁹ (Ohm x cm)⁻¹ ein Dunkelwiderstand von 10¹⁰ Ω und bei Lichtbestrahlung ein Hellwiderstand von 10⁵ Ω. Eine Kontaktierelektrode K₁ bis Kₙ hat bei der genannten Fläche von 10 µm x 10 µm durch das Elektrolyt E zur biologischen Zelle Ze einen Widerstand von etwa ebenfalls 10⁵ Ω, der durch die Helmholtz-Doppelschicht an der Grenzfläche Metall/Elektrolyt bestimmt wird. Es ergibt sich ein Gesamtübergangswiderstand von der biologischen Zelle Ze zur Anschlusselektrode A₁ bis Aₛ bei Lichtbestrahlung des lichtempfindlichen Elements P von etwa 2 x 10⁵ Ω. Ihm gegenüber beträgt der Gesamtübergangswiderstand bei dunklem lichtempfindlichem Element P etwa 10¹⁰ Ω. Es ergibt sich ein gutes Kontakt/Trenn-Verhältnis durch die hell/dunkel-Tastung der Mikroelektroden M₁ bis Mₙ zu ihrer Ansteuerung.

Da der Abstand zwischen den Mikroelektroden M₁ bis Mₙ groß gegenüber der Schichtdicke der lichtempfindlichen Schicht P ist, kann auf eine Isolierung der von ihr gebildeten lichtempfindlichen Elemente voneinander verzichtet werden und diese als durchgehende Schicht P ausgeführt sein, wie es beschrieben und dargestellt ist. Die Ansteuerung der Mikroelektroden M₁ bis Mₙ erfolgt bei dem in Figur 3 dargestellten Ausführungsbeispiel der Erfindung mittels eines in Zeilenrichtung, also quer zu den Anschlusselektroden A₁ bis Aₛ verlaufenden Lichtbalkens L, der die lichtempfindlichen Elemente in einer Zeile angeordneter Mikroelektroden M₁ bis Mₙ beleuchtet. Es werden also die Mikroelektroden M₁ bis Mₙ einer Zeile gleichzeitig angesteuert und die elektrischen Zellpotentiale der von diesen kontaktierten biologischen Zellen Ze über die Anschlusselektroden A₁ bis Aₛ abgeleitet oder diese biologischen Zellen elektrisch stimuliert. Der Lichtbalken L ist in Spaltenrichtung beweglich (Doppelpfeil in Figur 3). Die Ansteuerung kann selbstverständlich auch in verschiedenen Zeilen erfolgen, also nicht mittels eines Lichtbalkens, sondern mittels auf einzelne Mikroelektroden M₁ bis Mₙ gerichteter Lichtpunkte, wobei aus jeder Spalte nur eine Mikroelektrode M₁ bis Mₙ zu einem Zeitpunkt angesteuert werden kann. Ist der Abstand der Mikroelektroden M₁ bis Mₙ nicht ausreichend groß, so dass sich die Signale nebeneinander liegender Mikroelektroden M₁ bis Mₙ im vom Lichtbalken L beleuchteten und damit leitfähigen Bereich der lichtempfindlichen Schicht P gegenseitig beeinflussen, so kann kein durchgehender Lichtbalken L zur Ansteuerung der Mikroelektroden M₁ bis Mₙ Verwendung finden, es muss vielmehr zwischen den Mikroelektroden M₁ bis Mₙ stets ein dunkler Bereich verbleiben oder aber eine zusätzliche Isolatorschicht zwischen den Anschlusselektroden A₁ bis Aₛ in der lichtempfindlichen Schicht P angebracht sein (nicht dargestellt).

Bei einer Mikroelektrodenfläche von 10 µm x 10 µm und bei 20 µm Elektrodenabstand ergeben sich bei beispielsweise 60 Spalten mit jeweils 60 Mikroelektroden insgesamt 3600 Mikroelektroden M₁ bis Mₙ auf einem Substratfeld mit einer Fläche von 1,8 mm x 1,8 mm.

Bei der Mikroelektroden-Anordnung kann die Ansteuerung der lichtempfindlichen Elemente gegebenenfalls auch mit einer Leuchtdiodenmatrix als Substrat oder durch ein projiziertes Lichtbild erfolgen.

## Patentansprüche

1. Verwendung einer Mikroelektroden-Anordnung zur elektrischen Stimulation von Netzwerken biologischer Zellen, wobei die Mikroelektroden-Anordnung eine Vielzahl von Mikroelektroden (M₁ bis Mₙ) aufweist und wobei jede Mikroelektrode (M₁ bis Mₙ) eine Kontaktierelektrode (K₁ bis Kₙ), die mit dem Netzwerk biologischer Zellen (Ze) in elektrischen Kontakt bringbar ist, eine Anschlusselektrode (A; A₁ bis Aₛ), die elektrisch leitend mit einer Stimulationselektronik verbindbar ist, und ein lichtempfindliches Element (P), das zwischen der Kontaktierelektrode (K₁ bis Kₙ) und der Anschlusselektrode (A; A₁ bis Aₛ) angeordnet ist, aufweist, wobei das lichtempfindliche Element (P) als Schalter dient, der die Kontaktierelektrode (K₁ bis Kₙ) von der Anschlusselektrode (A; A₁ bis Aₛ) isoliert oder mit der Anschlusselektrode (A; A₁ bis Aₛ) verbindet, so dass jede Mikroelektrode (M₁ bis Mₙ) für sich durch Licht ansteuerbar ist.

2. Verwendung einer Mikroelektroden-Anordnung nach Anspruch 1, **dadurch gekennzeichnet, dass** die Kontaktierelektrode (K₁ bis Kₙ) und/oder das lichtempfindliche Element (P) und/oder die Anschlusselektrode (A; A₁ bis Aₛ) Dünnschichtelemente sind.

3. Verwendung einer Mikroelektroden-Anordnung nach Anspruch 1 oder 2, **dadurch gekennzeichnet, dass** das lichtempfindliche Element (P) als Dünnschicht-Fotowiderstand ausgebildet ist.

4. Verwendung einer Mikroelektroden-Anordnung nach einem der Ansprüche 1 bis 3, **dadurch gekennzeichnet, dass** sie eine gemeinsame Anschlusselektrode (A; A₁ bis Aₛ) für alle Mikroelektroden (M₁ bis Mₙ) oder für mehrere Mikroelektroden (M₁ bis Mₙ), die zu einer Gruppe von Mikroelektroden (M₁ bis Mₙ) zusammengefasst sind, aufweist.

5. Verwendung einer Mikroelektroden-Anordnung nach einem der Ansprüche 1 bis 4, **dadurch gekennzeichnet, dass** das lichtempfindliche Element (P) durchgehend über den Bereich aller oder mehrerer Mikroelektroden (M₁ bis Mₙ) ausgebildet ist.

6. Verwendung einer Mikroelektroden-Anordnung nach einem der Ansprüche 1 bis 5, **dadurch gekennzeichnet, dass** sie eine Lichtfaseroptik zur Ansteuerung ihrer Mikroelektroden (M₁ bis Mₙ) aufweist.

7. Verwendung einer Mikroelektroden-Anordnung nach Anspruch 6, **dadurch gekennzeichnet, dass** die Lichtfaseroptik eine Lichtfaser für jede Mikroelektrode (M₁ bis Mₙ) aufweist.

8. Verwendung einer Mikroelektroden-Anordnung nach Anspruch 7, **dadurch gekennzeichnet, dass** die Lichtfasern ein Substrat für die Mikroelektroden (M₁ bis Mₙ) bilden.

9. Verwendung einer Mikroelektroden-Anordnung nach Anspruch 7 oder 8, **dadurch gekennzeichnet, dass** die Lichtfaseroptik eine Lichtquelle für jede Lichtfaser aufweist.

10. Verwendung einer Mikroelektroden-Anordnung nach einem der Ansprüche 1 bis 9, **dadurch gekennzeichnet, dass** ein fokussierter Lichtstrahl örtlich begrenzt auf ein lichtempfindliches Element (P) einer oder mehrerer Mikroelektroden (M₁ bis Mₙ) gerichtet ist.

11. Verwendung einer Mikroelektroden-Anordnung nach Anspruch 1 bis 10, **dadurch gekennzeichnet, dass** die Ansteuerung mit einer Leuchtdiodenmatrix als Substrat oder durch ein projiziertes Lichtbild erfolgt.

12. Verwendung einer Mikroelektroden-Anordnung nach einem der Ansprüche 1 bis 11, **dadurch gekennzeichnet, dass** das lichtempfindliche Element (P) ein Helleiter ist.

13. Verwendung einer Mikroelektroden-Anordnung nach Anspruch 12, **dadurch gekennzeichnet, dass** das lichtempfindliche Element einen Dunkelwiderstand von näherungsweise 10¹⁰ Ω und einen Hellwiderstand von näherungsweise 10⁵ Ω aufweist.

14. Verwendung einer Mikroelektroden-Anordnung nach einem der Ansprüche 1 bis 13, **dadurch gekennzeichnet, dass** das lichtempfindliche Element (P) eine Dicke von 0,1 µm aufweist.

15. Verwendung einer Mikroelektroden-Anordnung nach einem der Ansprüche 1 bis 14, **dadurch gekennzeichnet, dass** das lichtempfindliche Element (P) amorphes Silizium aufweist.

16. Verwendung einer Mikroelektroden-Anordnung nach einem der Ansprüche 1 bis 15 als Retina-Implantat.

17. Mikroelektroden-Anordnung zum ortsaufgelösten Ableiten elektrischer Zellpotentiale oder zur elektrischen Stimulation von Netzwerken biologischer Zellen, mit einer Vielzahl von Mikroelektroden, wobei jede Mikroelektrode (M₁ bis Mₙ) eine Kontaktierelektrode (K₁ bis Kₙ), die mit dem Netzwerk biologischer Zellen (Ze) in elektrischen Kontakt bringbar ist, eine Anschlusselektrode (A; A₁ bis Aₛ), die elektrisch leitend mit einem Messgerät oder dgl. verbindbar ist und ein lichtempfindliches Element (P), das zwischen der Kontaktierelektrode (K₁ bis Kₙ) und der Anschlusselektrode (A; A₁ bis Aₛ) angeordnet ist, aufweist, und wobei vorzugsweise mehrere Mikroelektroden (M₁ bis Mₙ) zu einer Gruppe von Mikroelektroden (M₁ bis Mₙ) zusammengefasst sind, die eine gemeinsame Anschlusselektrode (A; A₁ bis Aₛ) aufweist, **dadurch gekennzeichnet, dass** das lichtempfindliche Element (P) als Dünnschicht-Fotowiderstand ausgebildet ist.

18. Mikroelektroden-Anordnung nach Anspruch 17, **dadurch gekennzeichnet, dass** die Kontaktierelektrode (K₁ bis Kₙ) und/ oder das lichtempfindliche Element (P) und/oder die Anschlusselektrode (A; A₁ bis Aₙ, A₁ bis Aₛ) Dünnschichtelemente sind.

19. Mikroelektroden-Anordnung nach Anspruch 17 oder 18, **dadurch gekennzeichnet, daß** sie eine gemeinsame Anschlusselektrode (A; A₁ bis Aₛ) für alle Mikroelektroden (M₁ bis Mₙ) oder für eine Gruppe von Mikroelektroden (M₁ bis Mₙ) aufweist.

20. Mikroelektroden-Anordnung nach einem der Ansprüche 17 bis 19, **dadurch gekennzeichnet, dass** das lichtempfindliche Element (P) durchgehend über den Bereich aller oder mehrerer Mikroelektroden (M₁ bis Mₙ) ausgebildet ist.

21. Mikroelektroden-Anordnung nach einem der Ansprüche 17 bis 20, **dadurch gekennzeichnet, dass** sie eine Lichtfaseroptik zur Ansteuerung ihrer Mikroelektroden (M₁ bis Mₙ) aufweist.

22. Mikroelektroden-Anordnung nach Anspruch 21, **dadurch gekennzeichnet, dass** die Lichtfaseroptik eine Lichtfaser für jede Mikroelektrode (M₁ bis Mₙ) aufweist.

23. Mikroelektroden-Anordnung nach Anspruch 22, **dadurch gekennzeichnet, dass** die Lichtfasern ein Substrat für die Mikroelektroden (M₁ bis Mₙ) bilden.

24. Mikroelektroden-Anordnung nach Anspruch 22 oder 23, **dadurch gekennzeichnet, dass** die Lichtfaseroptik eine Lichtquelle für jede Lichtfaser aufweist.

25. Mikroelektroden-Anordnung nach einem der Ansprüche 17 bis 24, **dadurch gekennzeichnet, dass** ein fokussierter Lichtstrahl örtlich begrenzt auf ein lichtempfindliches Element (P) einer oder mehrerer Mikroelektroden (M₁ bis Mₙ) gerichtet ist.

26. Mikroelektroden-Anordnung nach Anspruch 17, **dadurch gekennzeichnet, dass** die Ansteuerung mit einer Leuchtdiodenmatrix als Substrat oder durch ein projiziertes Lichtbild erfolgt.

27. Mikroelektroden-Anordnung nach einem der Ansprüche 17 bis 26, **dadurch gekennzeichnet, dass** das lichtempfindliche Element einen Dunkelwiderstand von näherungsweise 10¹⁰ Ω und einen Hellwiderstand von näherungsweise 10⁵ Ω aufweist.

28. Mikroelektroden-Anordnung nach einem der Ansprüche 17 bis 27, **dadurch gekennzeichnet, dass** das lichtempfindliche Element (P) eine Dicke von 0,1 µm aufweist.

29. Mikroelektroden-Anordnung nach einem der Ansprüche 17 bis 28, **dadurch gekennzeichnet, dass** das lichtempfindliche Element (P) amorphes Silizium aufweist.

30. Verwendung einer Mikroelektroden-Anordnung nach einem der Ansprüche 17 bis 29 als Implantat.
